# EUROPEAN PATENT SPECIFICATION

(11) **EP 0 316 745 B1**
(45) Date of publication and mention of the grant of the patent: **02.02.1994**
(21) Application number: 88118658.9
(22) Date of filing: 09.11.1988
(51) Int. Cl.: A61B 5/00

(54) **Liver function testing apparatus**
Vorrichtung zum Untersuchen der Leberfunktion
Dispositif pour examiner le fonctionnement du foie

(30) Priority: 13.11.1987 JP 287678/87
(43) Date of publication of application: 24.05.1989
(73) Proprietor: SUMITOMO ELECTRIC INDUSTRIES, LTD., Osaka-shi, Osaka 541 (JP)
(72) Inventor: Kanda, Masahiko c/o Osaka Works of Sumitomo, Konohana-ku Osaka (JP); Awazu, Kunio c/o Osaka Works of Sumitomo, Konohana-ku Osaka (JP)
(74) Representative: Patentanwälte Kirschner & Grosse

(56) References cited:
- EP-A- 0 276 477
- US-A- 3 677 648
- US-A- 4 017 192
- US-A- 4 602 641

## Description

### BACKGROUND OF THE INVENTION

### Field of the Invention

The present invention relates to a liver function testing apparatus, and more specifically, it relates to a liver function testing apparatus for automatically performing measurement for testing/diagnosing liver function by injecting specific color dye, which is selectively taken in and removed by only the liver, into blood and measuring a blood plasma disappearance rate and a retention rate thereof.

### Description of the Prior Art

In general, the blood plasma disappearance rate and the retention rate have been measured by a method of blood collection through use of indocyanine green (hereinafter referred to as ICG) serving as specific dye. According to this method, intravenous injection of ICG is given to a testee to perform blood collection three times after lapses of five, ten and 15 minutes from the injection, and blood serum is separated upon coagulation of a blood clot so that absorbance at a wavelength of 805 nm is measured through a spectrophotometer to obtain ICG concentration values in the blood serum after the lapses of five, ten and 15 minutes from a previously obtained calibration curve (corresponding ICG concentration in blood vs. absorbance), thereby to calculate the blood plasma disappearance rate and the retention rate. In recent years, widely employed is a method of changing the quantity of ICG injection to measure the blood plasma disappearance rate several times thereby to obtain index expressing the amount of hepatic cell function R_{MAX} (removal maximal).

Japanese Patent Publication Gazette No. 58649/1985 has already proposed a method of measuring the blood plasma disappearance rate and the retention rate without performing blood collection. According to this method, light is applied through the body surface of an organism, which in turn transmits light of a wavelength having high ICG absorption sensitivity and light of a wavelength having substantially no ICG absorption sensitivity. The respective quantities of transmitted light are measured to obtain the blood plasma disappearance rate and the retention rate from time change (dye disappearance curve) of the light quantities.

In the aforementioned first method of blood collection, it is necessary to correctly measure the blood collection time after injection. However, the time has not been accurately measured in an actual test, and the operation for measuring the index expressing the amount of hepatic cell function R_{MAX} has been complicated in adaptation to theory. Further, the testee has been subjected to heavy mental and physical burdens by blood collection. In addition, the index R_{MAX} measuring method of measuring the blood plasma disappearance rate several times by changing the quantity of ICG injection requires blood collection over ten times, whereby the burdens on the testee are further increased.

According to the second measuring method with no blood collection disclosed in Japanese Patent Publication Gazette No. 58649/1985, the output of a sensor actually attached to an organism is fluctuated by influence such as blood flow disturbance caused by suppression on a blood vessel, vibration of the organism, being the object of measurement, pulsation in the organism, change of blood volume in the vital tissue ( the blood volume in each part in the vital tissue is changed by merely vertically moving an arm) etc., whereby a correct dye disappearance curve cannot be obtained. Consequently, the blood plasma disappearance rate and the retention rate obtained by the curve cannot be recognized as being correct.

### SUMMARY OF THE INVENTION

Accordingly, a principal object of the present invention is to provide a liver function testing apparatus which can remove artifacts such as blood flow disturbance, vibration of an organism, pulsation in the organism and change of the blood volume in the organism, to enable correct measurement.

Briefly stated, vital tissue is exposed to first light of a wavelength absorbed by specific dye dosed into the blood of the vital tissue to be taken in and removed by the liver and second light of a wavelength not absorbed by the specific dye and first and second photoelectric conversion signals corresponding to the first light and the second light obtained from the vital tissue are sampled a plurality of times so that first and second coefficients of first and second regression line expressions between the first and second photoelectric conversion signals are decided on the basis of variable components in the blood included in the first and second photoelectric conversion signals sampled a plurality of times before and after injection of the specific dye and a value correlated with specific dye concentration in blood is operated on the basis of the sampling signals of a plurality of times in a prescribed period after injection of the specific dye and the decided coefficients of the first and second regression line expressions to obtain a coefficient of a simulation function as a function of time on the basis of the said value through the method of least squares, thereby to obtain a blood plasma disappearance rate of the specific dye and a retention rate of the specific dye in a prescribed period.

Thus, according to the present invention, correct time management of disappearance curves of the specific dye is enabled to obtain correct data. Further, the blood plasma disappearance rate and the retention rate can be obtained not from several samples along the conventional blood correction method but from a large number of data of the disappearance curves, thereby to improve reliability of the data.

In a preferred embodiment of the present invention, the second coefficient is decided within a prescribed period upon injection of the specific dye in an arbitrary short period after a time when the specific dye is uniformly distributed in the blood.

Further, first constants A₁ and B₁ are obtained by performing regression line analysis in accordance with the following operation expression:

$\text{logCL₁ = A₁·logCL₂ + B₁}$

assuming that CL₁ and CL₂ represent average values of first and second photoelectric conversion signals sampled a plurality of times before injection of the specific dye.

Second constants A₂ and B₂ are obtained by performing regression line analysis in accordance with the following operation expression:

$\text{logCL₁′ = A₂·logCL₂′ + B₂}$

assuming that CL₁′ and CL₂′ represent average values of the first and second photoelectric conversion signals sampled a plurality of times after a lapse of a prescribed period upon injection of the specific dye, while

$\text{logL₁₀ = (A₁·B₂ - A₂·B₁)/(A₁ - A₂)}$

is obtained as logL₁₀.

These and other objects, features, aspects and advantages of the present invention will become more apparent from the following detailed description of the present invention when taken in conjunction with the accompanying drawings.

### BRIEF DESCRIPTION OF THE DRAWINGS

Figs. 1 to 4 are diagrams for illustrating the principle of biocalibration employed in the present invention;
Fig. 5 is a schematic block diagram showing the entire structure of an embodiment of the present invention;
Fig. 6 is a timing chart for detecting quantities of light of wavelengths λ₁ and λ₂ after passage through a prescribed optical path in a measured object;
Fig. 7 illustrates data stored in a RAM as shown in Fig. 1;
Figs. 8A to 8D are flow charts for concretely illustrating the operation of the embodiment, in which

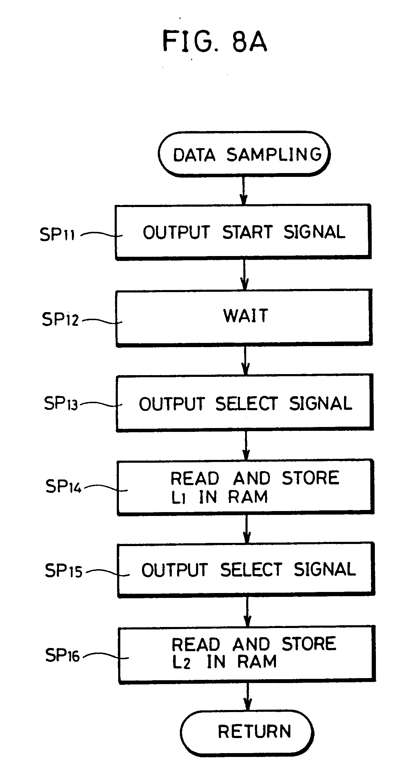

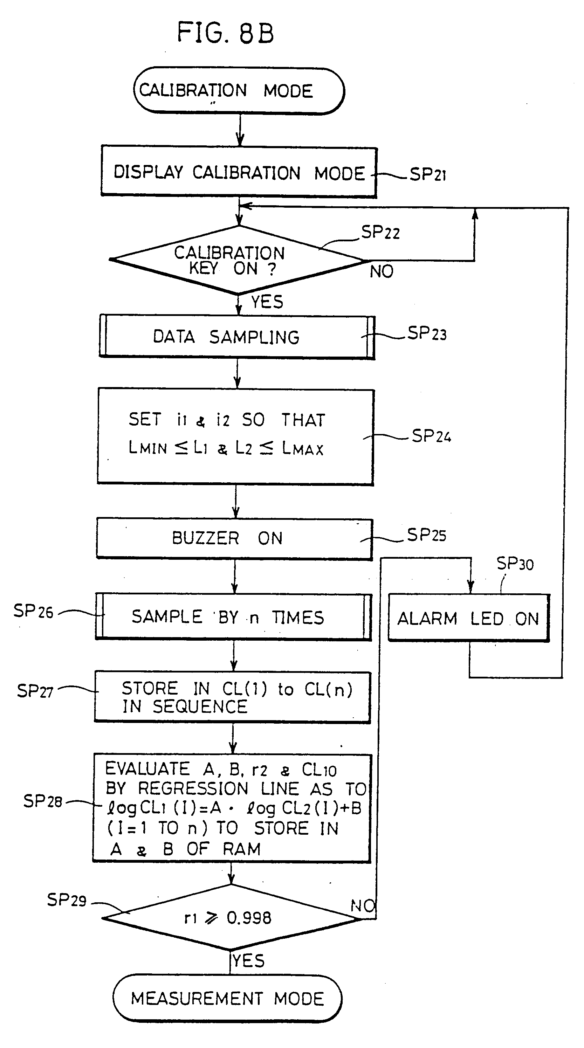

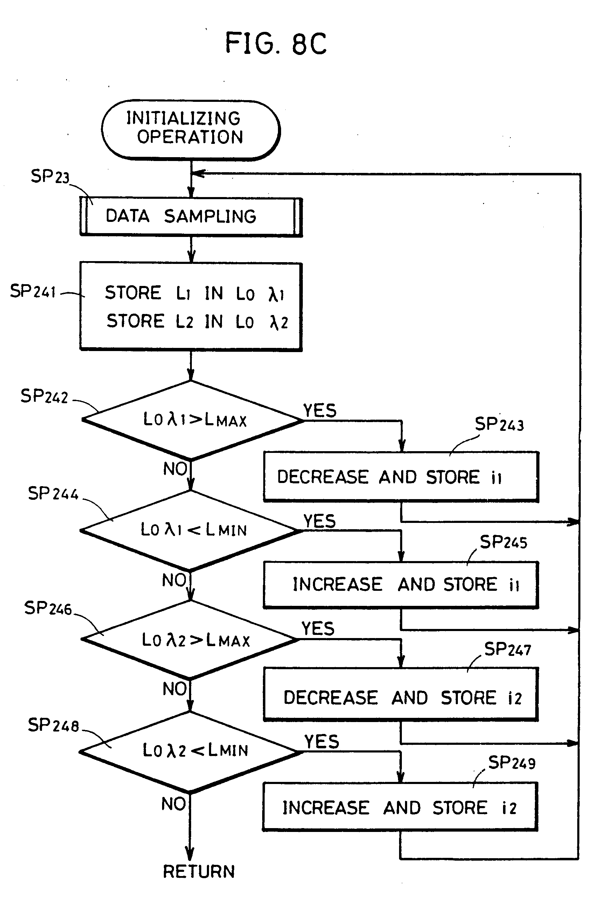

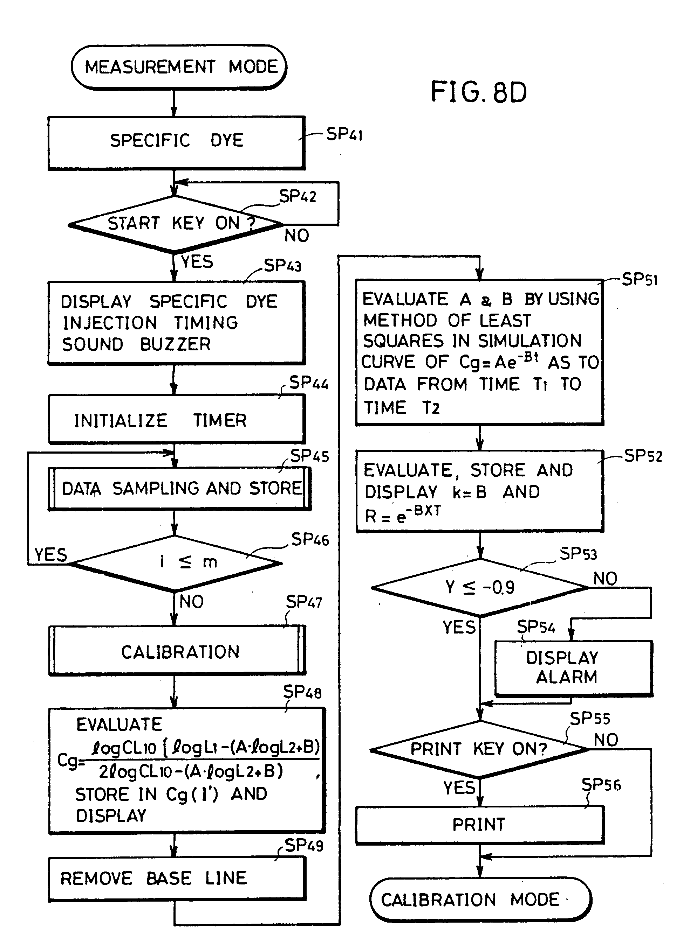

Fig. 8A shows a data sampling subroutine, Fig. 8B shows a biocalibration mode, Fig. 8C shows an initialization mode and Fig. 8D shows a measurement mode;

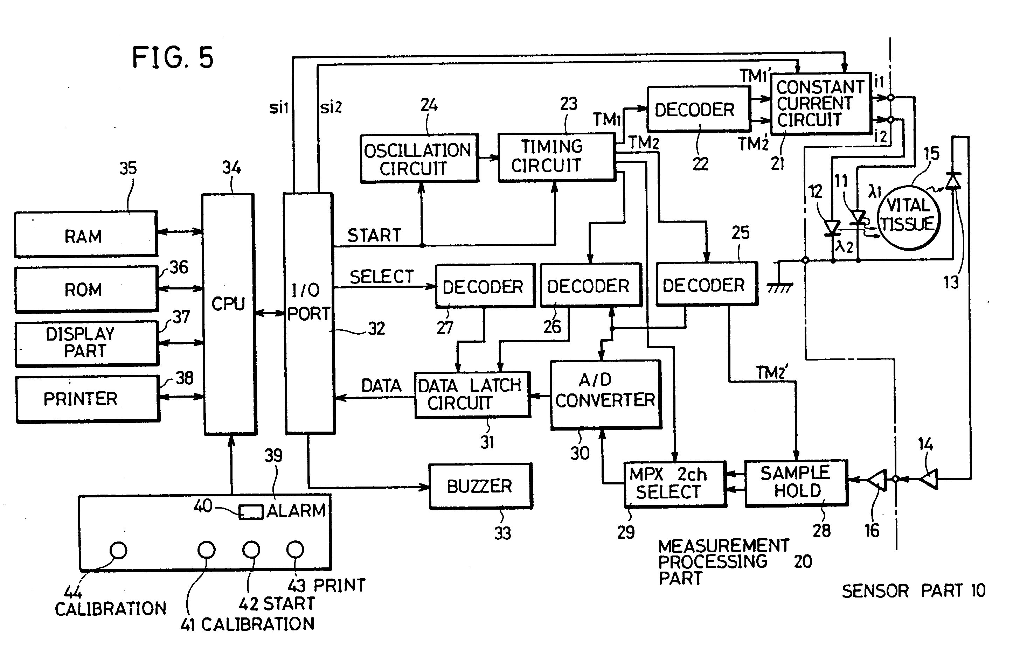

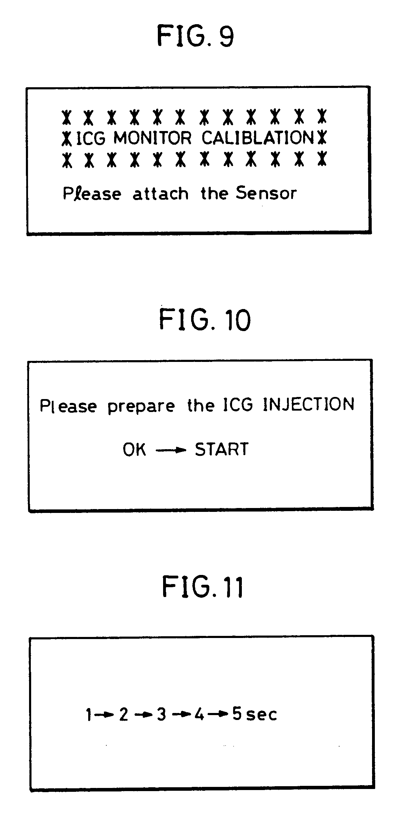

Figs. 9 to 12 are illustrative of exemplary displays on a display part as shown in Fig. 5;

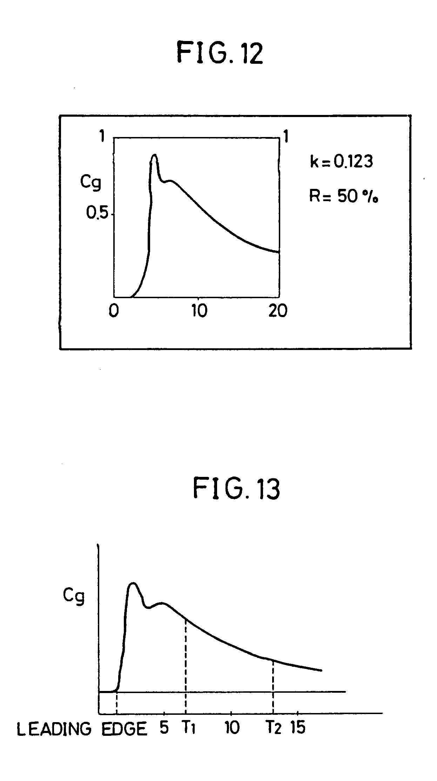

Fig. 13 shows an example of a disappearance curve of specific dye measured in the present invention;

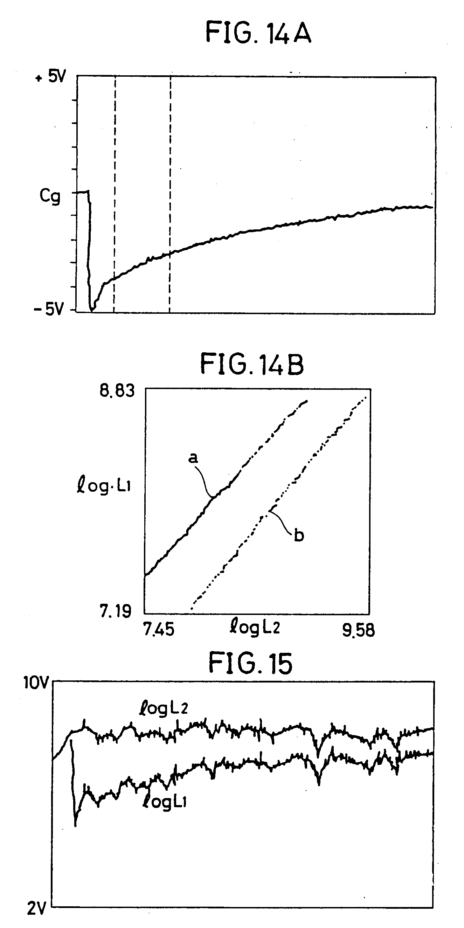

Fig. 14A illustrates relation between a disappearance curve, a blood plasma disappearance rate and a 15-minute retention rate measured through the present invention;
Fig. 14B illustrates values L₁ and L₂ measured in the present invention and calibration curves of two times; and
Fig. 15 illustrates values L₁ and L₂ measured in the present invention.

### DESCRIPTION OF THE PREFERRED EMBODIMENTS

Before explaining embodiments of the present invention, description is now made on the principle of biocalibration employed in the present invention.

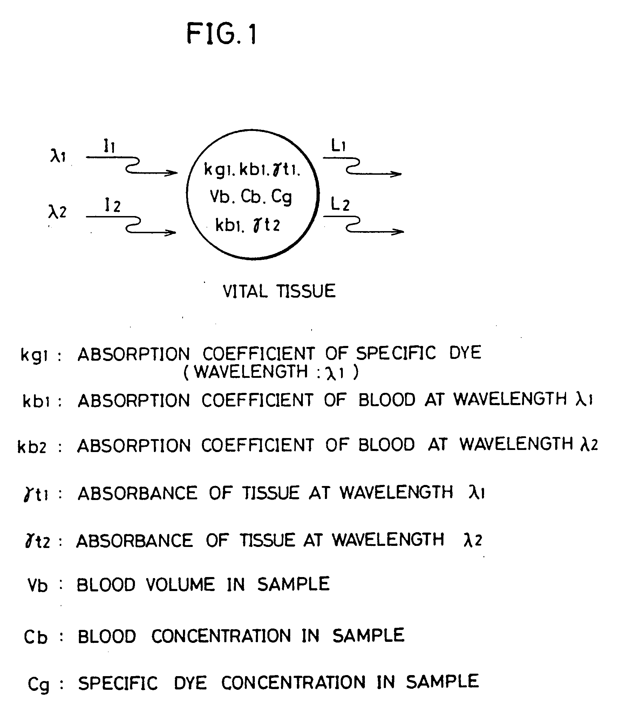

Figs. 1 to 4 are diagrams for illustrating the principle of the biocalibration in the present invention.

It is assumed that symbols I₁ and I₂ indicate quantities of light having a wavelength λ₁ which is largely absorbed by specific dye and light of a wavelength λ₂ which is not absorbed by the specific dye incident upon vital tissue, and symbols L₁ and L₂ indicate light quantities after passage through a prescribed optical path in the vital tissue. Relations between the incident light quantities I₁ and I₂ and the passing light quantities L₁ and L₂ in injection of the specific dye are as follows:

$\text{(1) logI₁/L₁ = kg₁·Cg·Vb + f₁(Cb, Vb) + γt₁}$

$\text{(2) logI₂/L₂ = f₂(Cb, Vb) + γt₂}$

Respective coefficients and variables are shown in Fig. 1. Symbols f₁ and f₂ represent functions which are determined by characteristics of blood at the wavelengths λ₁ and λ₂.

On the other hand, relations between the incident light quantities I₁ and I₂ and the passing light quantities L₁ and L₂ before injection of the specific dye are as follows:

$\text{(3) logI₁/L₁ = f₁(Cb, Vb) + γt₁}$

$\text{(4) logI₂/L₂ = f₂(Cb, Vb) + γt₂}$

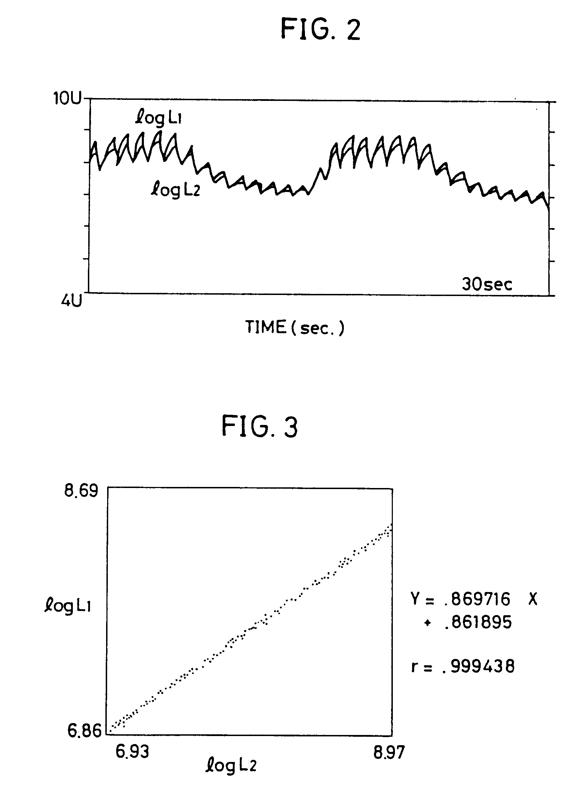

The relation between the passing light quantities L₁ and L₂ previous to actual injection of the specific dye is measured as shown in Fig. 2, to be in the linear relation shown in Fig. 3. This is the data in case of attaching a sensor to an organism and fluctuating the blood volume in the organism. It has been confirmed that such linearity has reproducibility, with no individual difference.

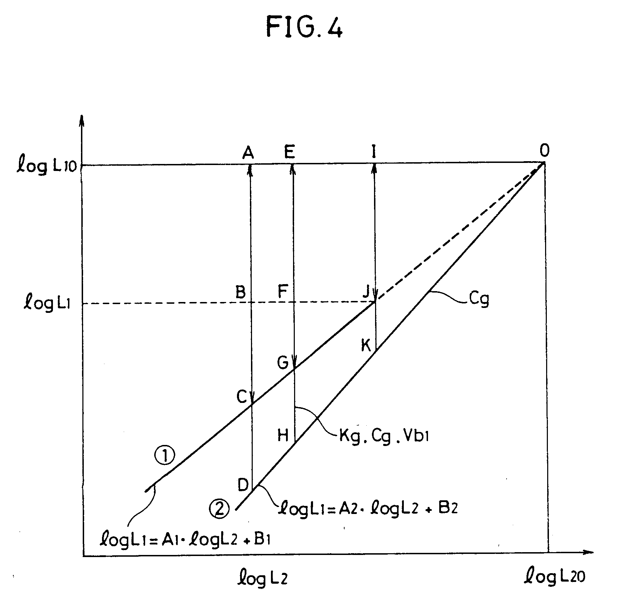

Then, the expressions (3) and (4) would appear as follows, as expressed by a straight line ① in Fig. 4:

$\text{(5) logL₁ = A₁logL₂ + B₁}$

That is, the same can be expressed as follows, by using the expressions (3) and (4):

$\text{(6) logI₁ - {f₁(Cb, Vb) + γt₁} =A[logI₂ - {f₂(Cb, Vb) + γt₂}] +B}$

where Cb represents blood concentration in a sample and Vb represents blood volume in the sample.

A function C obtained by multiplying concentration of the specific dye by the blood volume in the sample and the absorption coefficient of the specific dye by using the expressions (1) and (2) after injection of the specific dye can be expressed as follows:

$\text{(7) C = logL₁ - [A·logL₂ + B]}$

The function C is found by the expression (7) as follows:

$\text{(8) C = logI₁ - kg·Cg·Vb - f₁(Cb, Vb) + γt₁ - A[logI₂ - {f₂(Cb, Vb) + γt₂}] - B}$

Through the expression (6), we have:

$\text{(9) C = - kg·Cg·Vb}$

Hence, it is understood that a signal of the function C can be obtained by using Fig. 3 as a calibration curve.

As to the function C, however, although the coefficient kg is constant, it can be considered that the blood volume Vb in each part is changed from time to time, and hence, if the blood volume Vb in a prescribed sample created by the sensor once attached is changed, the amount of the specific dye is also changed in proportion thereto although the dye concentration remains unchanged. This is typically shown in Fig. 4.

Referring to Fig. 4, a straight line ① represents a calibration curve before injection of the specific dye, while another straight line ② represents a calibration curve in an arbitrary short period after injection of the specific dye. The straight line ② performs calibration in a short period, and hence the specific dye is believed to be constant. The straight line ②, considered similarly to the expression (5), is expressed as follows:

$\text{logL₁ = A₂·logL₂ + B₂}$

The intersection 0 of the straight lines ① and ② is considered as an ischemia point, which is expressed as follows:

$\text{logL₁₀ = (A₁·B₂ - A₂·B₁)/(A₁ - A₂)}$

In Fig. 4,

$\text{GH/CD = OG/OC = OE/OA = EG/AC, and}$
$\text{GH/EG = CD/AC}$

hence,

$\text{kg·Cg·Vb₁/Vb₁ = kg·Cg·Vb₂/Vb₂}$

Thus,

$\text{GH/EC = kg·Cg = CD/AC}$

whereby concentration in blood can be measured.

Normalizing as Y-axis logL₁₀ of the intersection 0 between the straight lines ① and ②, the blood volume Vb is expressed as follows: 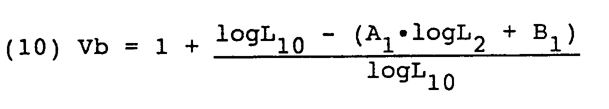
Hence, a signal Cg corresponding to the specific dye concentration can be found by the expressions (7) and (10) as follows: 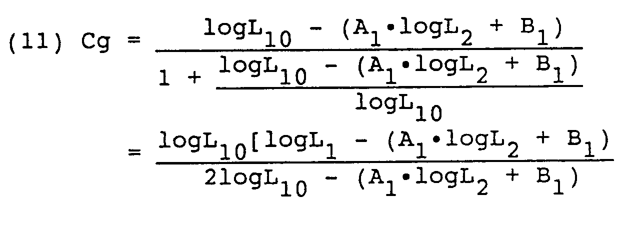
Using the method of least squares, the function Cg of a simulation curve in time change of the aforementioned result Cg of calculation is expressed as follows:

${\text{(12) Cg = Ae}}^{\text{Bt}}$

where t represents the elapsed time after injection of the specific dye and symbols A and B represent constants.

The constants A and B are found by the above expression (12). The blood plasma disappearance rate k and the T-minute retention rate R % are expressed as follows:

$\text{(13) k = -B}$

${\text{(14) R % = e}}^{\text{BT}}$

where T represents the elapsed time after injection characteristically expressing intake of the specific dye into the liver.

While the biocalibration employed in the present invention has been described in the above, description is now made on an embodiment of the present invention employing the aforementioned biocalibration.

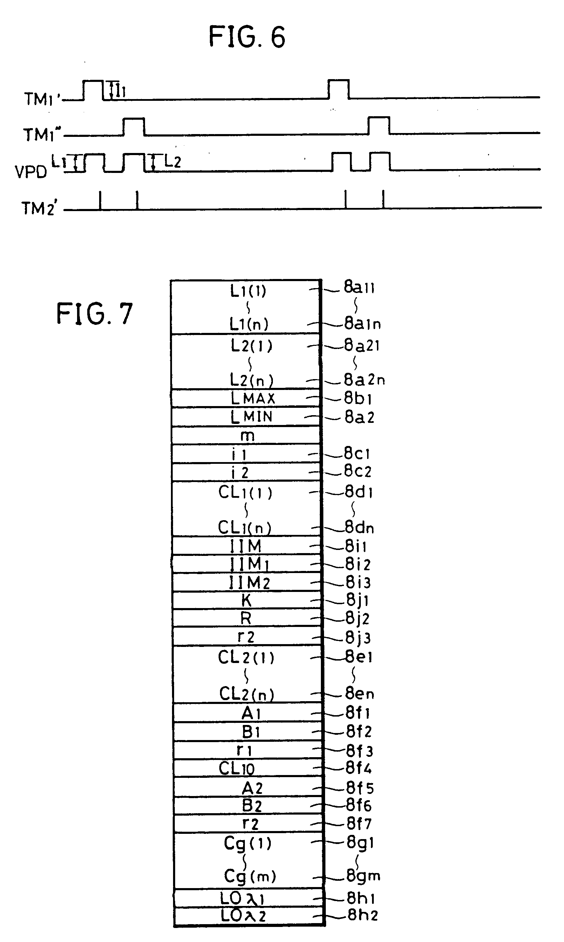

Fig. 5 is a schematic block diagram showing an embodiment of the present invention, Fig. 6 is a timing chart for detecting quantities of light of wavelengths λ₁ and λ₂ after passage through a prescribed optical path in a measured object, and Fig. 7 illustrates data stored in a RAM as shown in Fig. 5.

Referring to Fig. 5, a liver function testing apparatus is formed by a sensor part 10 and a measurement processing part 20. The sensor part 10 includes a first light source 11, a second light source 12, a light receiving element 13 and a preamplifier 14. The first light source 11 and the second light source 12 generate optical pulses of a wavelength λ₁ having large absorbance to specific dye and optical pulses of a wavelength λ₂ having no absorbance, respectively. The light receiving element 13 receives light applied to vital tissue 15 from the light sources 11 and 12 to pass through a prescribed optical path. The light sources 11 and 12 are driven by the measurement processing part 20 to alternately emit light by pulse operation, respectively.

The measurement processing part 20 includes a CPU 34 which serves as arithmetic means. The CPU 34 supplies a start signal to an oscillation circuit 24 and a timing circuit 23 through an I/O port 32. The oscillation circuit 24 regularly oscillates to produce a prescribed clock signal. This clock signal and the aforementioned start signal are utilized to supply constant currents i₁ and i₂ to the first light source 11 and the second light source 12 from a constant current circuit 21 through the timing circuit 23 and a decoder 22 at timing TM₁′ and TM₁'' in Fig. 6.

The light emitted from the first light source 11 and the light emitted from the second light source 12 pass through the prescribed optical path in the vital tissue 15, to be incident upon the light receiving element 13. A current generated from the light receiving element 13 is supplied to the preamplifier 14 to be subjected to current-to-voltage conversion, while being amplified to be supplied to the measurement processing part 20. Output of the preamplifier 14 is amplified to a level within a prescribed range by an amplifier 16 provided in the measurement processing part 20, whereby output such as V_{PD} in Fig. 6 is obtained. A sample and hold circuit 28 samples and holds output from the amplifier 16 on the basis of a timing signal TM₂′, shown in Fig. 6, generated by the timing circuit 23 and a decoder 25.

The signal thus sampled and held is selected by a multiplexer 29 and converted into a digital signal by an A-D converter 30, to be data-latched by a data latch 31. At this time, the multiplexer 29, the A-D converter 30 and the data latch 31 are controlled in timing by the timing circuit 23 and the decoder 26.

The latched data are timed by a decoder 27 through a select signal outputted from the CPU 34 through the I/O port 32, to be taken in a RAM 35 as digital signals L₁ and L₂. The I/O port 32 is connected with a buzzer 33, which informs timing for injecting the specific dye. Further, the CPU 34 is connected with the RAM 35, a ROM 36, a display part 37 and an operation part 28. The RAM 35 is adapted to store data as shown in Fig. 7 as hereinafter described, and the ROM 36 stores programs based on flow charts shown in Figs. 8A to 8D as hereinafter described. The display part 37 displays data as shown in Figs. 9 to 12, as hereinafter described. A printer 38 is adapted to print the result of a liver function test.

A function part 39 includes an alarm LED 40, first and second calibration keys 41 and 44, a start key 42 and a print key 43. The alarm LED 40 is adapted to display an alarm when reliability of the test result is small and the first calibration key 41 is adapted to set a first biocalibration mode before injection of specific dye, while the second calibration key 44 is adapted to set a second biocalibration mode after injection of the specific dye. The start key 42 is adapted to command start of a measurement mode and the print key 43 is adapted to command printout of the test result.

In the aforementioned exemplary structure shown in Fig. 5, the light emitted from the first and second light sources 11 and 12 to pass through the prescribed optical path in the vital tissue 15 is received by a single light receiving element 13. However, such means is not restricted to this but light receiving elements may be provided in correspondence to the first and second light sources 11 and 12 respectively to sample outputs of the respective light receiving elements, thereby to read the respective sampling outputs by the CPU 34 in a time-sharing manner. Alternatively, a single light source commonly emitting light having a wavelength λ₁ absorbed by specific dye and light having a wavelength λ₂ not absorbed by the same may be provided as light source means, with provision of two filters for individually transmitting the light of the respective wavelengths and light receiving elements corresponding to the respective ones of the filters.

Fig. 7 illustrates data stored in the RAM 35 as shown in Fig. 5 and Figs. 8A to 8D are flow charts for illustrating concrete operation of the embodiment of the present invention, while Figs. 9 to 12 are illustrative of exemplary displays on the display part shown in Fig. 5, Fig. 14 is illustrative of an exemplary disappearance curve of specific dye, and the blood plasma disappearance rate k and the T-minute retention rate R % measured in the present invention.

With reference to Figs. 5, 8A to 8D and 14, description is now made on the concrete operation of the embodiment of the present invention.

The operation of the inventive apparatus includes a data sampling mode, first and second biocalibration modes, an initialization mode and a measurement mode, and Figs. 8A, 8B, 8C and 8D show operation flows in these modes respectively.

First, it is pointed out that the data sampling mode shown in Fig. 8A is executed as subroutines in the biocalibration modes and the measurement mode as hereinafter described. Steps (abbreviated as SP in the figures) SP11 to SP16 are adapted to quantities of light of a pair of wavelengths λ₁ and λ₂ after passage through a measured object and store the same in the RAM 35. Namely, the CPU 34 outputs the start signal from a line shown in Fig. 5 through the I/O port 23 at the step SP11. The values L₁ and L₂ are data-latched by the start signal, as hereinabove described. The CPU 34 waits until the data are latched at the step SP12.

Then, at the step SP13, the CPU 34 outputs the select signal to a select line shown in Fig. 5 through the I/O port 32, to read the data of L₁ through the I/O port 32 at the step SP14, thereby to store the same in a storage area 8a1 of the RAM 35 as shown in Fig. 7.

Similarly, the CPU 34 stores the data of L₂ in a storage area 8a2 of the RAM 35 at the steps SP15 and SP16.

Upon completion of the aforementioned operation at the step SP16, the CPU 34 returns to the original step. This will be described with reference to Fig. 8B showing the biocalibration mode and Fig. 8D showing the measurement mode.

Fig. 8B shows the operation flow chart of the first biocalibration mode, which is started upon power supply to the apparatus or upon completion of the operation of the measurement mode shown in Fig. 8D, as hereinafter described. At a step SP21, the CPU 34 makes the biocalibration mode appear on the display part 37. This display shows that the apparatus enters the biocalibration mode and indicates mounting of the sensor part 10 shown in Fig. 9, for example. In accordance with this indication, an operator attaches the sensor part 10 to the vital tissue 15.

Thereafter the CPU 34 waits until the calibration key 41 is operated at a step SP22. When the calibration key 41 is operated, the CPU 34 advances to a step SP23, to execute the data sampling subroutine shown in Fig. 8A, as hereinabove described.

Then, the CPU 34 controls the constant current circuit 21 as shown in Fig. 5 so that the data L₁ and L₂ read at the step SP23 are within ranges of light quantity data L_{MAX} and L_{MIN} stored in storage areas 8b1 and 8b2 of the RAM 35. The CPU 34 then stores current set values i₁ and i₂ in storage areas 8c1 and 8c2 in the RAM 35. Thereafter the currents i₁ and i₂ regularly flow to the light sources 11 and 12. Initializing operation for the aforementioned currents will be described in further detail with reference to Fig. 8C.

Then, the CPU 34 sounds the buzzer at a step SP25, to inform that initialization is completed. Subsequent steps SP26 to SP29 are shown as a flow chart for performing the aforementioned biocalibration. In more concrete terms, the CPU 34 samples the values of L₁ and L₂ n times respectively at the steps SP26 and SP27, to make CL₁(1) to CL₁(n) stored in storage areas 8d1 to 8dn and CL₂(1) to CL₂(n) stored in storage areas 831 to 83n. At the subsequent step SP28, the CPU 34 performs regression line analysis with respect to logCL₁(I) and logCL₂(I) (I = 1 to n), in accordance with the following operation expression:

$\text{logCL₁(I) = A₁·logCL₂(I) + B₁}$

The CPU 34 finds the values A₁ and B₁ in the above operation expression, a correlation coefficient r₁ and the maximum value of CL₁(I) (I = 1 to n) as CL₁₀, to store the same in storage areas 8f1, 8f2, 8f3 and 8f4 in the RAM 35 respectively.

Then, at the step SP29, the CPU 34 determines whether or not the correlation coefficient r₁ is at least 0.998 in order to verify reliability of the biocalibration, advances to a step SP30 if the same is less than 0.998 to light the alarm LED 40, and returns to the step SP22 to again perform biocalibration. On the other hand, if a determination is made that the correlation coefficient r₁ is at least 0.998, the CPU 34 advances to the measurement mode as shown in Fig. 8D. The reference value 0.998 of the correlation coefficient r₁ herein employed is a mere example, which is determined by performance of the entire apparatus. During the data sampling of n times at the step SP26, the testee raises and brings down his hand and suppresses the same by the sensor, in order to change the blood volume in the organism.

With reference to Fig. 8C, the aforementioned initializing operation at the step SP24 as shown in Fig. 8B will now be described in more concrete terms.

The light quantity data L₁ and L₂ of the light of the wavelengths λ₁ and λ₂ are stored in the storage areas 8a1 and 8a2 of the RAM 35. At a step SP241, the CPU 34 makes the values of L₁ and L₂ stored in storage areas 8h1 and 8h2 in the RAM 35 as L0λ₁ and L0λ₂ respectively. Then the CPU 34 executes steps SP242 to SP249, to adjust the set values of the currents flowing from the constant current circuit 21 so that L0λ₁ and L0λ₂ are set between the light quantity data L_{MAX} and L_{MIN} (L_{MAX} > L_{MIN}) stored in the storage areas 8b1 and 8b2 of the RAM 35.

In more concrete terms, if L0λ₁ is greater than L_{MAX} at the step SP242, the CPU 34 advances to the step SP243 to set the current set value i₁ at a small value to again execute the steps SP23 and SP241, and a determination is again made as to whether or not L0λ′₁ is greater than L_{MAX} at the step SP242. If L0λ₁ is less than L_{MAX′} the CPU 34 advances to the step SP244 to determine whether or not L0λ₁ is less than L_{MIN}. If L0λ₁ is less than L_{MIN}, the CPU 34 increases the value of the current set value i₁ at the step SP245, to return to the aforementioned step SP23. This operation is repeated to set the current set value i₁ so that L0λ₁ is between L_{MAX} and L_{MIN}.

Then, at the steps SP246 to SP249, the current set value i₂ is set so that L0λ₂ is between L_{MAX} and L_{MIN}, similarly to the steps SP242 to SP245. Thus, the current set values i₁ and i₂ finally set at the steps SP23 to SP249 are stored in the storage areas 8c1 and 8c2 of the RAM 35.

With reference to Fig. 8D, description is now made on the measurement mode. At a step SP41, the CPU 34 makes a display for injection of the specific dye on the display part 37. As to this display, indication is made for injection of the specific dye such as ICG as shown in Fig. 10, for example. In accordance with the display, the operator prepares for injection of the specific dye to the testee. At a step SP42, the CPU 34 waits until the start key 42 is operated. Upon a determination that the start key 42 is operated, the CPU 34 displays timing for injecting the specific dye at a step SP43, while sounding the buzzer 33. This is displayed as 1 → 2 → 3 → 4 → 5 as shown in Fig. 11, for example, so that the measurer injects the specific dye upon display of "5". The CPU 34 generates a first sound from the buzzer 33 with the displays of "1", "2", "3" and "4", while generating a different sound from the buzzer 33 upon display of "5".

Upon generation of the sound and the display, the measurer injects the specific dye. The CPU 34 sets "0" as the initial value of a timer at a step SP44. Then, at a step SP45, the CPU 34 executes a data sampling program, which is the subroutine as hereinabove described with reference to Fig. 8A. Then, the sampling data are stored in the storage areas 8a11 to 8a1n and 8a21 to 8a2n of the RAM 35 as L₁(1) to L₁(n) and L₂(n) to L₂(n), respectively. Assuming that m represents the sampling number of the disappearance curves of the specific dye, I represents an integer of 1 to m, and one sampling time is expressed as $\text{ITM = TS/(m - 1)}$ 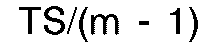 assuming that Ts represents a measuring time of the disappearance curve. This coincides with the injection time of the specific dye if I = 1, as a matter of course. The CPU 34 determines whether or not i is greater than m at a step SP46 and returns to the step SP45 if i is less than m, to repeat sampling and storage. Upon a determination that i is greater than m, the CPU 34 advances to a step SP47 and performs second biocalibration similarly to the above description with reference to Fig. 8B, to obtain constants A₂ and B₂ and a correlation function r₂ and store the same in the storage areas 8f5, 8f6 and 8f7 of the RAM 35 respectively. As a matter of course, the second biocalibration is not restricted to the step SP47, but may be performed at the steps SP45 and SP46 in a uniformly distributed state of ICG concentration in blood after injection of the ICG through the calibration key 44 shown in Fig. 5.

At a step SP48, the CPU 34 performs operation based on the following operation expression by using the constants A₁, B₁, A₂ and B₂ obtained in the first and second biocalibration modes as hereinabove described and stored in the storage areas 8f1, 8f2, 8f3, 8f5 and 8f6 of the RAM 35 to store Cg(I) in a storage area 8g1 of the RAM 35: 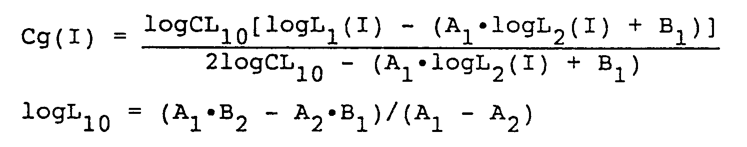
The value of Cg(I) is displayed on the display part 37 at the step SP48 in a mode shown in Fig. 12, for example. Referring to Fig. 12, the axis of abscissa indicates the elapsed time from injection of the specific dye and the axis of ordinate indicates the value of Cg(I). The data Cg(I) stored in the storage areas 8g1 to 8gm of the RAM 35 draw a disappearance curve of the specific dye as shown in Fig. 13, for example, and the leading edge thereof is detected so that data preceding the same are subtracted as baselines from the respective values of Cg(I) at a step SP49, to be again stored in the storage areas 8g1 to 8gm. Needless to say, data sampling times T₁ to T₂ at the step SP45 may be average values of k times, in order to improve the accuracy of measurement.

Then, at a step SP51, the CPU 34 finds the constants A and B by using the method of least squares in a simulation curve of:

${\text{Cg(I) = Ae}}^{\text{Bt}}$
$\text{I = Ts/(m - 1) (min.)}$

with respect to data between times T₁ to T₂ (0 < T₁ < T₂ < Ts) within the data Cg(I) stored in the storage areas 8g1 to 8gm.

Then, the CPU 34 performs operation of the blood plasma disappearance rate $\text{k = -B}$ and the T-minute retention rate ${\text{R % = e}}^{\text{BT}}$ at a step SP52, to evaluate k and R %. The values k and R % thus evaluated are stored in storage areas 8j1 and 8j2 of the RAM 35, respectively. At this time, the CPU 34 operates a correlation coefficient r₂ by the method of least squares, to make the correlation coefficient r₂ thus operated stored in a storage area 8j3 of the RAM 35. The CPU 34 further generates an end sound from the buzzer 33.

Further, the CPU 34 makes the values k and R % appear on the display part 26 in a mode shown in Fig. 12, for example. Then, at a step SP53, the CPU 34 determines whether or not the correlation coefficient r₂ is less than 0.95, for example. This determination is made to check the degree of correlation since correlation is improved as the correlation coefficient r₂ approaches -1. The value -0.95 is provisionally selected between zero and -1, and reliability of the apparatus is improved as the value comes closer to -1.

If the correlation coefficient r₂ is greater than 0.95, for example, the CPU 34 determines that reliability is insufficient to light the alarm LED 40 at the step SP54. On the other hand, if the correlation coefficient r₂ is less than -0.95, for example, at the step SP53, the CPU 34 advances to a step SP55 without flashing the alarm LED 44, since the measurement is reliable. At the step SP55, the CPU 34 determines whether or not the print key 43 is operated, to make the printer 38 print the values k and R % if the determination is of YES.

If necessary, the CPU 34 makes characteristic dye disappearance curves of Cg(I) stored in the storage areas 8g1 to 8gn of the RAM 35 printed, to advance to the first biocalibration mode shown in Fig. 8B. Also when a determination is made that the print key 43 is not operated at the step SP55, the CPU 34 advances to the first biocalibration mode.

Fig. 14B illustrates calibration curves of two times in the experiment, in which the curve a is that before injection and the curve b is a calibration curve after a prescribed period.

In the experiment shown in Fig. 14A, the sensor part 10 was attached on a left fingertip of a male patient having hepatic disease (age: 66, weight: 48 Kg), to intravenously inject an aqueous solution containing 24 mg of ICG (0.5 mg per Kg) from the vein of his right front elbow. Fig. 15 shows the time change of L₁ and L₂ in case of employing a light emitting diode of wavelength λ₁ = 810 nm as the first light source 11 and a light emitting diode of wavelength λ₂ = 940 nm as the second light source 12.

The value k calculated by the ICG disappearance curve was 0.09 as shown in Fig. 14A and the value R % was 24.1 %, while the value k measured by the conventional blood collection method was 0.099 and the value R % was 22.6 %, substantially in coincidence. Fig. 15 also shows raw data of L₁ and L₂. It is clearly understood from Fig. 14B that the blood volume in the organism was fluctuated.

The value k obtained through the present invention can be extended to an apparatus for obtaining and calculating values k of various ICG doses.

According to the present invention as hereinabove described, vital tissue is exposed to first light of a wavelength absorbed by specific dye dosed into the blood of the vital tissue to be taken in and removed by the liver and second light of a wavelength not absorbed by the specific dye and first and second photoelectric conversion signals corresponding to the first light and the second light obtained from the vital tissue are sampled a plurality of times so that first and second coefficients of first and second regression line expressions between the first and second photoelectric conversion signals are decided on the basis of variable components in the blood included in the sampled first and second photoelectric conversion signals before and after injection of the specific dye to perform biocalibration, thereby to obtain the blood plasma disappearance rate of the specific dye and the retention rate on the basis of a plurality of sampling outputs during a prescribed period after injection of the specific dye, the decided coefficients of the regression line expressions and prescribed operation expressions. Thus, correct time management of the disappearance curve of the specific dye is enabled, to obtain correct data. Further, the blood plasma disappearance rate and the retention rate can be obtained not from several samples along the conventional blood collection method but from a large number of disappearance curve data, to improve reliability of the data.

## Claims

1. A liver function testing apparatus for testing liver function, including
light source means (11, 12) for exposing vital tissue to first light of a wave length absorbed by specific dye dosed into blood of said vital tissue to be taken in and removed by the liver and second light of a wave length not absorbed by said specific dye; and
photoelectric conversion means (13) for outputting first and second photoelectric conversion signals corresponding to said first light and said second light applied to said vital tissue by said light source means and obtained from said vital tissue characterised in that the apparatus comprises;
sampling means (28) for sampling said first and second photoelectric conversion signals a plurality of times for testing liver function in accordance with output from said sampling means before and after injection of said specific dye; said testing apparatus comprising:
first arithmetic operation means (34, SP28) for obtaining first constants A₁ and B₁ by performing the regression line analysis in accordance with the following operation expression:
$\text{log CL₁ = A₁· logCL₂ + B₁}$
assuming that CL₁ and CL₂ represent values of said first and second photoelectric conversion signals sampled by said sampling means a plurality of times before injection of said specific dye; second arithmetic operation means (34, SP28, SP51) for obtaining second constants A₂ and B₂ by performing the regression line analysis in accordance with the following operation expression:
$\text{logCL₁' = A₂·logCL₂' + B₂}$
assuming that CL₁' and CL₂' represent values of said first and second photoelectric conversion signals sampled by said sampling means a plurality of times after injection of said specific dye;
an arithmetic operation means (34, SP48, SP51, SP52) for obtaining, on the basis of the constants A₁, B₁, A₂ and B₂ obtained by said first and second arithmetic operation means, a value logL₁₀ in accordance with the following operation expression:
$\text{logL₁₀ = (A₁·B₂ - A₂·B₁)/(A₁ - A₂)}$
obtaining, on the basis of said obtained constants A₁, B₁ and L₁₀, a value Cg correlated with said specific dye concentration in accordance with the following operation expression: 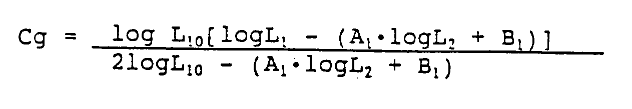 assuming that t represents a prescribed period after injection of said specific dye,
obtaining constants A and B by using the method of least squares on the basis of a simulation curve in accordance with the following operation expression:
${\text{Cg = Ae}}^{\text{Bt}}$
and obtaining a blood plasma disappearance rate k and retention rate R% in said time t in accordance with the following equations:
$\text{k = -B}$
${\text{R% = e}}^{\text{Bt}}$

2. A liver function testing apparatus in accordance with claim 1, wherein
said second arithmetic operation means (SP28) obtains said second constant within a prescribed period upon injection of said specific dye in an arbitrary short period after a time when said specific dye is uniformly distributed in said blood.

3. A liver function testing apparatus in accordance with claim 1, wherein
said arithmetic operation means includes means for operating a correlation coefficient of said regression line expression,
said liver function testing apparatus further including informing means (33) for giving an alarm when said correlation coefficient operated by said means for operating said correlation coefficient is greater than a predetermined value.

4. A liver function testing apparatus in accordance with claim 1, wherein
said arithmetic operation means includes means for operating a correlation coefficient of said simulation function,
said liver function testing apparatus further including informing means (33) for giving an alarm when said correlation coefficient of said simulation function is greater than a predetermined value.

5. A liver function testing apparatus in accordance with claim 1, further including mode selection means (41, 42) for selecting a biocalibration mode for performing operation for deciding said coefficient of said regression line expression by said decision means and a measurement mode for performing operation for operating said value correlated with said specific dye concentration by said arithmetic means.

6. A liver function testing apparatus in accordance with claim 5, further including means (34) for activating said decision means in response to selection of said biocalibration mode by said mode selection means.

7. A liver function testing apparatus in accordance with claim 1, further including means (34) for activating said arithmetic means in response to selection of said measurement mode by said mode selection means.

8. A liver function testing apparatus in accordance with claim 1, further including set means (SP241 - SP249) for setting intensity levels of said first light and said second light emitted from said light source means so that levels of said first and second photoelectric conversion signals are within a predetermined range.

## Patentansprüche

1. Leberfunktionsuntersuchungsvorrichtung zur Untersuchung der Leberfunktion, umfassend:
- Lichtquelleneinrichtungen (11, 12), um lebendes Gewebe erstem Licht einer Wellenlänge, die von einem bestimmten Farbstoff, der dosiert in das Blut des lebenden Gewebes eingebracht wird, um von der Leber aufgenommen und entfernt zu werden, absorbiert wird, und zweitem Licht einer Wellenlänge, die nicht durch den bestimmten Farbstoff absorbiert wird, auszusetzen; und
- photoelektrische Umwandlungseinrichtungen (13) zum Abgeben von ersten und zweiten photoelektrischen Umwandlungsignalen, die dem durch die Lichtquelleneinrichtungen auf das lebende Gewebe angewandten und von dem lebenden Gewebe erhaltenen ersten und zweiten Licht entsprechen,
**dadurch gekennzeichnet, daß** die Vorrichtung enthält:
- Erfassungseinrichtungen (28), um die ersten und zweiten photoelektrischen Umwandlungssignale eine Anzahl von Malen zu erfassen, um die Leberfunktion gemäß den Ausgangswerten von der Erfassungseinrichtung vor und nach dem Injizieren des bestimmten Farbstoffs zu untersuchen; wobei die Untersuchungseinrichtung aufweist:
- erste Rechenarbeitseinrichtung (34, SP28), um erste Konstanten A₁ und B₁ durch Durchführen einer Regressionslinienanalyse nach dem folgenden Arbeitsausdruck:
$\text{log CL₁ = A₁·logCL₂ + B₁}$
zu erhalten, wobei angenommen wird, daß CL₁ und CL₂ Werte der durch die Erfassungseinrichtung eine Anzahl von Malen vor dem Injizieren des bestimmten Farbstoffs erfaßten ersten und zweiten photoelektrischen Umwandlungssignale sind,
- zweite Rechenarbeiseinrichtung (34, SP28, SP51), um zweite Konstanten A₂ und B₂ durch Durchführen einer Regressionslinienanalyse nach dem folgenden Arbeitsausdruck:
$\text{log CL₁' = A₂·logCL₂' + B₂}$
zu erhalten, wobei angenommen wird, daß CL₁' und CL₂' Werte der durch die Erfassungseinrichtung eine Vielzahl von Malen nach dem Injizieren des bestimmten Farbstoffs erfaßten ersten und zweiten photoelektrischen Umwandlungssignale sind,
- eine Rechenarbeitseinrichtung (34, SP28, SP51, SP52), um auf der Grundlage der durch die ersten und zweiten Rechenarbeitseinrichtungen erhaltenen Konstanten A₁, B₁, A₂ und B₂ einen Wert logL₁₀ gemäß dem folgenden Arbeitsausdruck:
$\text{logL₁₀ = (A₁·B₂ - A₂·B₁)/(A₁ - A₂)}$
zu erhalten, und um so auf der Grundlage der erhaltenen Konstanten A₁, B₁ und L₁₀ einen mit der Konzentration des bestimmten Farbstoffs in Korrelation stehenden Wert Cg gemäß dem folgenden Arbeitsausdruck 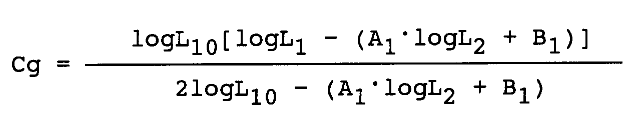 zu erhalten, wobei angenommen wird, daß t eine vorgegebene Zeitspanne nach Injizieren des bestimmten Farbstoffs ist,
- wobei man die Konstanten A und B durch Verwenden des Verfahrens der kleinsten Quadrate auf der Grundlage einer Simulationskurve nach dem folgenden Arbeitsausdruck enthält:
${\text{Cg = Ae}}^{\text{Bt}}$
- und wobei man einen Blutplasmaschwundgrad k und einen Zurückhaltegrad R% in der Zeit t gemäß den folgenden Gleichungen erhält:
$\text{k = -B}$
${\text{R% = e}}^{\text{Bt}} \text{.}$

2. Leberfunktionsuntersuchungsvorrichtung nach Anspruch 1, **dadurch gekennzeichnet, daß** die zweite Rechenarbeitseinrichtung (SP28) die zweite Konstante innerhalb einer bestimmten Zeitspanne nach Injizieren des bestimmten Farbstoffs in einer beliebig kurzen Zeitspanne nach der Zeit, wenn der bestimmte Farbstoff gleichmäßig in dem Blut verteilt ist, zu errechnen.

3. Leberfunktionsuntersuchungsvorrichtung nach Anspruch 1, **dadurch gekennzeichnet, daß**
- die Rechenarbeitseinrichtung eine Einrichtung zum Berechnen eines Korrelationskoeffizienten des Regressionslinienausdrucks umfaßt,
- die Leberfunktionsuntersuchungsvorrichtung weiterhin eine Informationseinrichtung (33) umfaßt, um einen Alarm zu geben, wenn der durch die Einrichtung zum Berechnen des Korrelationskoeffizienten errechnete Korrelationskoeffizient größer als ein vorgegebener Wert ist.

4. Leberfunktionsuntersuchungsvorrichtung nach Anspruch 1, **dadurch gekennzeichnet, daß**
- die Rechenarbeitseinrichtung eine Einrichtung zum Errechnen eines Korrelationskoeffizienten der Simulationsfunktion umfaßt,
- die Leberfunktionsuntersuchungsvorrichtung weiterhin eine Informationseinrichtung (33) umfaßt, um einen Alarm zu geben, wenn der Korrelationskoeffizient der Simuationsfunktion größer als ein vorgegebener Wert ist.

5. Leberfunktionsuntersuchungsvorrichtung nach Anspruch 1, welche weiterhin eine Modusauswahleinrichtung (41, 42) zum Auswählen eines Bio-Eichmodus, um eine Berechnung zur Bestimmung des Koeffizienten des Regressionslinienausdrucks durch die Entscheidungseinrichtung durchzuführen, und eines Meßmodus, um den Arbeitsvorgang zur Berechnung des mit der Konzentration des bestimmten Farbstoffs in Korrelation stehenden Wertes durch die Recheneinrichtung durchzuführen, umfaßt.

6. Leberfunktionsuntersuchungsvorrichtung nach Anspruch 5, welche weiterhin Einrichtungen (34) zur Aktivierung der Entscheidungseinrichtung in Reaktion auf das Anwählen des Bio-Eichmodus durch die Modusauswahleinrichtung umfaßt.

7. Leberfunktionsuntersuchungsvorrichtung nach Anspruch 1, welche weiterhin eine Einrichtung (34) zum Aktivierung der Recheneinrichtung in Reaktion auf das Anwählen des Meßmodus durch die Modusauswahleinrichtung umfaßt.

8. Leberfunktionsuntersuchungsvorrichtung nach Anspruch 1, welche weiterhin eine Einstelleinrichtung (SP241 - SP249) umfaßt, um die Intensitätspegel des von den Lichtquelleneinrichtungen abgegebenen ersten und zweiten Lichts so einzustellen, daß die Pegel der ersten und zweiten photoelektrischen Umwandlungssignale innerhalb eines vorgegebenen Bereichs liegen.

## Revendications

1. Dispositif de contrôle du fonctionnement du foie, pour examiner le fonctionnement du foie, incluant :
- des moyens de source de lumière (11, 12) pour exposer du tissu vital à une première lumière, d'une longueur d'onde absorbée par un colorant spécifique dosé dans le sang dudit tissu vital devant pénétrer dans et être éliminé par le foie, et à une seconde lumière, d'une longueur d'onde non absorbée par ledit colorant spécifique; et
- des moyens de conversion photoélectrique (13) pour sortir des premier et second signaux de conversion photoélectrique correspondant à ladite première lumière et à ladite seconde lumière appliquées audit tissu vital par lesdits moyens de source de lumière et obtenus à partir dudit tissu vital;
caractérisé en ce que le dispositif comprend :
- des moyens d'échantillonnage (28) pour échantillonner lesdits premier et second signaux de conversion photoélectrique une pluralité de fois, pour examiner le fonctionnement du foie suivant la sortie desdits moyens d'échantillonnage avant et après l'injection dudit colorant spécifique; ledit dispositif de contrôle comprenant :
- des premiers moyens de calcul arithmétique (34, SP28) pour obtenir des premières constantes A₁ et B₁ en effectuant une analyse par régression linéaire selon l'expression mathématique suivante :
$\text{logCL₁ = A₁.logCL₂ + B₁}$
supposant que CL₁ et CL₂ représentent les valeurs desdits premier et second signaux de conversion photoélectrique échantillonnés par lesdits moyens d'échantillonnage une pluralité de fois avant l'injection dudit colorant spécifique;
- des seconds moyens de calcul arithmétique (34, SP28, SP51) pour obtenir des secondes constantes A₂ et B₂ en effectuant une analyse par régression linéaire selon l'expression mathématique suivante :
$\text{logCL₁' = A₂.logCL₂' + B₂}$
en supposant que CL₁' et CL₂' représentent les valeurs desdits premier et second signaux de conversion photoélectrique échantillonnés par lesdits moyens d'échantillonnage une pluralité de fois après l'injection dudit colorant spécifique;
- des moyens de calcul arithmétique (34, SP48, SP51, SP52) pour obtenir, sur la base des constantes A₁, B₁, A₂ et B₂ obtenues par lesdits premiers et seconds moyens de calcul arithmétique, une valeur logL₁₀ selon l'expression mathématique suivante :
$\text{logL₁₀ = (A₁.B₂ - A₂.B₁)/(A₁ - A₂)}$
pour obtenir, sur la base desdites constantes A₁, B₁ obtenues et de L₁₀, une valeur Cg en corrélation avec la concentration dudit colorant spécifique selon l'expression mathématique suivante : 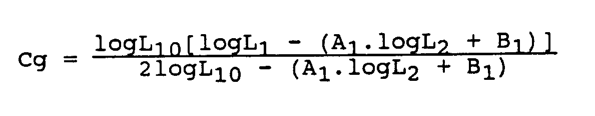 en supposant que t représente une période prescrite après l'injection dudit colorant spécifique,
pour obtenir des constantes A et B en utilisant la méthode des moindres carrés sur la base d'une courbe de simulation selon l'expression mathématique suivante :
${\text{Cg = Ae}}^{\text{Bt}}$
et pour obtenir une vitesse de disparition dans le plasma sanguin, k, et un taux de rétention, R%, dans ledit temps t selon les équations suivantes :
$\text{k = -B}$
${\text{R% = e}}^{\text{Bt}} \text{.}$

2. Dispositif de contrôle du fonctionnement du foie selon la revendication 1, dans lequel :
- lesdits seconds moyens de calcul arithmétique (SP28) obtiennent lesdites secondes constantes en une période prescrite lors de l'injection dudit colorant spécifique, une courte période arbitraire après le moment où ledit colorant spécifique est distribué uniformément dans ledit sang.

3. Dispositif de contrôle du fonctionnement du foie selon la revendication 1, dans lequel :
- lesdits moyens de calcul arithmétique incluent des moyens pour calculer un coefficient de corrélation de ladite expression de régression linéaire,
- ledit dispositif de contrôle du fonctionnement du foie inclut de plus des moyens d'information (33) pour donner l'alarme lorsque ledit coefficient de corrélation, calculé par lesdits moyens pour calculer ledit coefficient de corrélation, est supérieur à une valeur prédéterminée.

4. Dispositif de contrôle du fonctionnement du foie selon la revendication 1, dans lequel :
- lesdits moyens de calcul arithmétique incluent des moyens pour calculer un coefficient de corrélation de ladite fonction de simulation,
- ledit dispositif de contrôle du fonctionnement du foie inclut de plus des moyens d'information (33) pour donner l'alarme lorsque ledit coefficient de corrélation de ladite fonction de simulation est supérieur à une valeur prédéterminée.

5. Dispositif de contrôle du fonctionnement du foie selon la revendication 1, incluant de plus des moyens de sélection de mode (41, 42) pour sélectionner un mode de bioétalonnage, pour effectuer des calculs pour décider dudit coefficient de ladite expression de régression linéaire par lesdits moyens de décision, et un mode de mesure, pour effectuer des calculs pour calculer ladite valeur en corrélation avec la concentration dudit colorant spécifique par lesdits moyens arithmétiques.

6. Dispositif de contrôle du fonctionnement du foie selon la revendication 5, incluant de plus des moyens (34) pour activer lesdits moyens de décision en réponse à la sélection dudit mode de bioétalonnage par lesdits moyens de sélection de mode.

7. Dispositif de contrôle du fonctionnement du foie selon la revendication 1, incluant de plus des moyens (34) pour activer lesdits moyens arithmétiques en réponse à la sélection dudit mode de mesure par lesdits moyens de sélection de mode.

8. Dispositif de contrôle du fonctionnement du foie selon la revendication 1, incluant de plus des moyens de définition (SP241-SP249) pour définir des niveaux d'intensité de ladite première lumière et de ladite seconde lumière émises à partir desdits moyens de source de lumière de telle sorte que les niveaux desdits premier et second signaux de conversion photoélectrique sont compris dans un domaine prédéterminé.
